# EUROPEAN PATENT APPLICATION

(11) **EP 3 072 477 A1**
(43) Date of publication of application: **28.09.2016**
(21) Application number: 16466005.2
(22) Date of filing: 21.03.2016
(51) Int. Cl.: A61F 2/20

(54) **A VOCAL CORD SUBSTITUTION AND A METHOD OF TUNING THE VOCAL CORD SUBSTITUTION**

(30) Priority: 23.03.2015 CZ 20150201
(71) Applicant: CVUT V Praze, Fakulta Strojní, 166 07 Praha 6 (CZ); Ústav termomechaniky AV CR, v.v.i., 18200 Praha 8 (CZ); Univerzita Karlova v Praze, 12800 Praha 2 (CZ)
(72) Inventor: Vampola, Tomás, 27354 Lidice (CZ); Horácek, Jaromir, 10800 Praha 10 (CZ); Dusková Smrcková, Jaroslava, 16200 Praha 6 (CZ); Köhler, Jakub, 41201 Litomerice (CZ); Klepácek, Ivo, 14900 Praha 4 (CZ)
(74) Representative: Novotny, Karel

(57) **Abstract**

The invention concerns an artificial vocal fold (1) consisting of two parts, in the transversal direction the two parts are slidingly placed in opposite walls of guide frame (5) and in the longitudinal direction they are attached to attachment wall (9) of guide frame (5) on one side and to attachment wall (9) of setting frame (6) on the opposite side, whereas inner area (4) of artificial vocal fold (1) is connected to a pressure fluid source.

A method of tuning the vocal cord substitution as described in the invention lies in that the basic frequency of the human vocal fold is determined, on the basis of which an initial prestressing of the artificial vocal fold is created by setting-up a distance of guide frames (5) and setting frames (6) enclosing the artificial vocal folds, followed by changing vibration characteristics of the artificial vocal fold through changing the fluid pressure in the artificial vocal fold, the vibration characteristics are monitored and compared to the demanded vibration characteristics of the human vocal fold, until similarity of the compared vibration characteristics of the artificial and human vocal fold is achieved.

## Description

### Technical Field of the Invention

The invention concerns a vocal cord substitution comprising an artificial vocal fold and a method of tuning the vocal cord substitution.

### Technical Field of the Invention

At present, four basic approaches are applied in an endeavour for voice reconstruction after devastating damage of vocal cord tissues.
- The electrolarynx is used to produce speech
   The electrolarynx voice is created by placing the device to the throat under the mandible; the result is a technical non-personal sound, which is of a low intensity and not easily understandable.
- Esophageal speech training
   The esophageal voice is produced by vibrating the upper segment of the vocal tract by forcing the air from the stomach or by inletting the air into the esophagus through an artificially created channel between the trachea and the esophagus (plastic surgery of the larynx, transplantation or creating an opening in the preserved parts of the larynx).
- Surgery methods facilitating the voice rehabilitation
   Since 1874 a metal tracheostomic cannula with an additional arm to the lower part of the larynx has been known. This procedure cannot be functional for a long time due to infections.
- Voice prosthesis
   At present human vocal cord replicas have been known, which can be used for research of basic physical phenomena related to generation of sound as a result of vibrations of vocal cords. There are replicas functioning on a principle of reed musical instruments, but there are also models of vocal cords made of plastic materials (e.g. polyurethane, silicone), which are similar to human vocal cords in geometry and dimensions, or PROVOX prostheses, which are relatively self-cleaning, but not producing the voice. Sounds can be coarsely produced without using hands through a silicone withdrawal pipe.

The aim of this invention is a method and a device for a vocal cord substitution being frequency tunable within the entire normal human voice (speech) range according to the individual patient dispositions and a possibility of its use not only for the human vocal cords function research on models, as so far, but also for implantation of this substitution in vivo after an excision of this organ from the human body, for example in consequence of a malignant tumor.

### Subject Matter of the Invention

The subject matter of the vocal cord substitution according to this invention consists in a two-piece artificial vocal fold; in the transversal direction the two parts are slidingly placed in opposite sides of a guide frame and in the longitudinal direction they are attached to an attachment wall of the guide frame on one side and to an attachment wall of the guide frame on the opposite side, whereas an inner area of the artificial vocal fold is connected to a pressure fluid source. In the guide frame or a setting frame, there is an adjusting nut arranged into which an adjusting bolt is engaging. The artificial vocal fold is fitted on its ends in the longitudinal direction with attachment elements for attaching to the attachment walls of the guide frame and the setting frame. The attachment walls of the guide frame and the setting frame are sectional. The artificial vocal fold is fitted with an outer layer and a middle layer enveloping an inner area. The guide frame and/or setting frame are/is fitted with attachment elements. The guide frame and the setting frame are of rounded prism-shapes whereas their attachment walls are equipped with straight-through holes.

The subject matter of the method of tuning the vocal fold substitution comprising an artificial vocal fold according to the invention lies in that the basic frequency of the human vocal fold is determined, on the basis of which an initial prestressing of the artificial vocal fold is created by setting a distance of the frames enclosing the artificial vocal fold, followed by changing vibration characteristics of the artificial vocal fold through changing the fluid pressure in the artificial vocal fold; the vibration characteristics are monitored and compared to the demanded vibration characteristics for the human vocal fold and after achieving the similarity of the compared vibration characteristics of the artificial and human vocal fold, the vocal cord substitution is tuned for its application.

The vocal cord substitutions as described in this invention were subjected to thorough experimental tests, which proved their functionality in a range of physiological parameters of the normal human phonation, i.e. airflow rate and pressure of the air inlet by the trachea from lungs to vocal cords, generating a sufficient level of a primary sound needed for its frequency modification and propagation by the vocal tract to the human mouth. These vocal cord substitutions can also be functional at the lowest possible pressure and flow rate of air allowing continuous speech and not straining the lungs during the voice production too much. The guide frame and the setting frame are of proper shapes in accordance with a way of the use of the vocal cord substitution.

The vocal cord substitution is also easily replaceable and it is possible to be inserted primarily, i.e. immediately following the total laryngectomia, or secondarily, i.e. whenever later after the surgical wound has healed.

### Overview of Figures in Drawings

The Figures show examples of embodiments of the device for the frequency tunable vocal cord substitution
Fig. 1 A profile of a part of the artificial vocal fold
Fig. 2 and Fig. 3 An embodiment of the vocal cord substitution
Fig. 4 and Fig. 5 Another embodiment of the vocal cord substitution
Fig. 6 and Fig. 7 An embodiment of the vocal cord substitution for implementation of the vocal cord substitution in vivo in a place of the tracheal annulus
Fig. 8 to Fig. 10 An embodiment of the vocal cord substitution for implementation of the vocal cord substitution in vivo in a tracheostomic cannula

### Examples of the technical solution embodiments

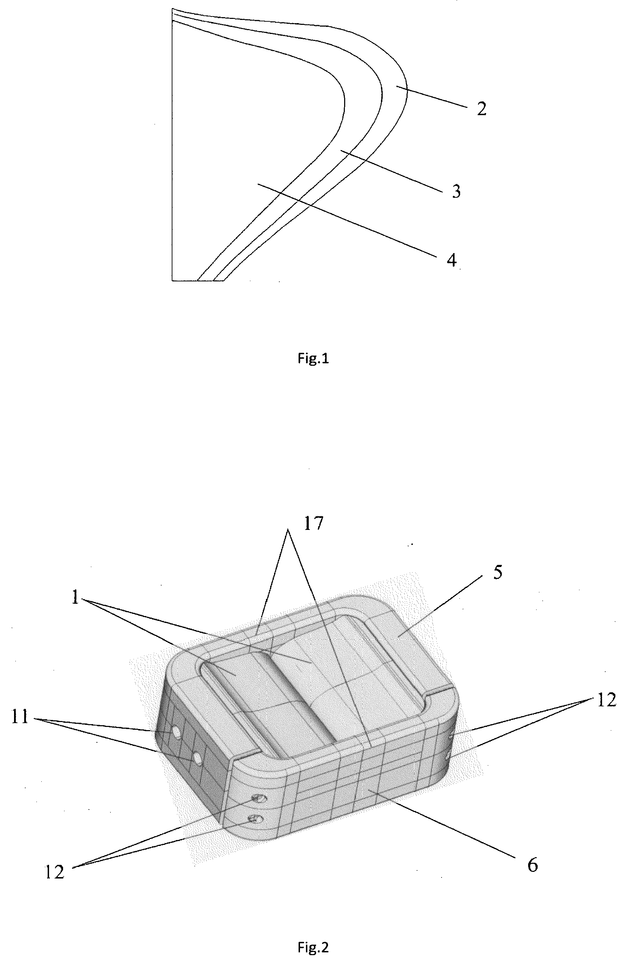

Fig. 1 shows a cross section of one part of artificial vocal fold 1, which is inserted along with the second part to the vocal cord substitution depicted in Figures below. Artificial vocal fold 1 is fitted with an outer boundary layer 2 defining the physiological profile of the vocal fold obtained from images acquired from the computed tomography investigation. Middle layer 3 features different material properties; this layer is dominating in influencing the artificial vocal fold vibration character. Area 4 represents a volume filled with viscous fluid. The resulting vibration character can be affected by the overpressure value of this fluid.

Fig. 2 shows one embodiment of the vocal cord substitution for an experimental verification of phonation properties in vitro, related from the human vocal cord structure on the basis of autopsy findings, CT scans and MRI displays. Two parts of artificial vocal fold 1, the cross section of which is depicted in Fig. 1, are in the transversal direction placed slidingly in two opposite walls of guide frame 5, and in the longitudinal direction they are attached to the attachment wall of guide frame 5 on one side and to the attachment wall of setting frame 6 on the other side. Guide frame 5 and setting frame 6 are mutually adjustable in the longitudinal direction of artificial vocal fold 1. Setting frame 6 is slidingly placed in guide frame 5. Adjusting bolts 12 and adjusting nuts 13 are used for adjusting guide frame 5 and setting frame 6 relatively to one another; adjusting bolts 12 are placed in setting frame 6 and adjusting nuts 13 in guide frame 5, or vice versa. By turning adjusting bolts 12 engaged in adjusting nuts 13 the attachment walls of guide frame 5 get farer or nearer from/to the attachment wall of setting frame 6, thus changing the length of artificial vocal fold 1 in the longitudinal direction. The basic phonation characteristic of the vocal cord substitution is achieved by this.

The walls of guide frame 5 are equipped with filling openings 11 for fluid supplied into artificial vocal fold 1, so that the pressure change in the artificial vocal fold can be achieved. To facilitate the attachment of artificial vocal fold 1, guide frame 5 and setting frame 6 are fitted with removable walls 17, which, after inserting artificial vocal fold 1 into frames 5 and 6, are connected to the matching fixed walls, thus fixing the attachment of artificial vocal fold 1, or more precisely its attachment elements, in the frame walls.

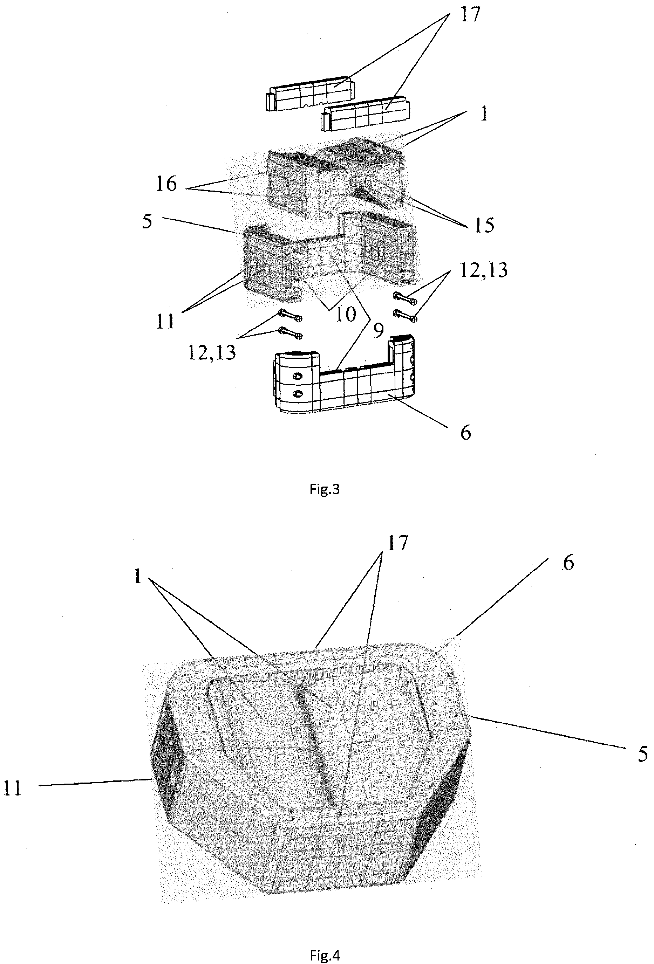

Fig. 3 shows a disassembled structure of the vocal cord substitution depicted in Fig. 2. A dovetail joint for pushing guide elements 16 of artificial vocal fold 1 in place is created in the body of guide frame 5, allowing a free motion of the vocal cord substitution in the longitudinal direction. In attachment walls 9 of guide frame 5 and setting frame 6, shaped slots are created, enabling inserting attachment elements 15 of artificial vocal fold 1. In the transversal direction of artificial vocal fold 1, in the walls of guide frame 5, filling openings 11 are arranged, enabling to modify the pre-stressing pressure value in the body of artificial vocal fold 1.

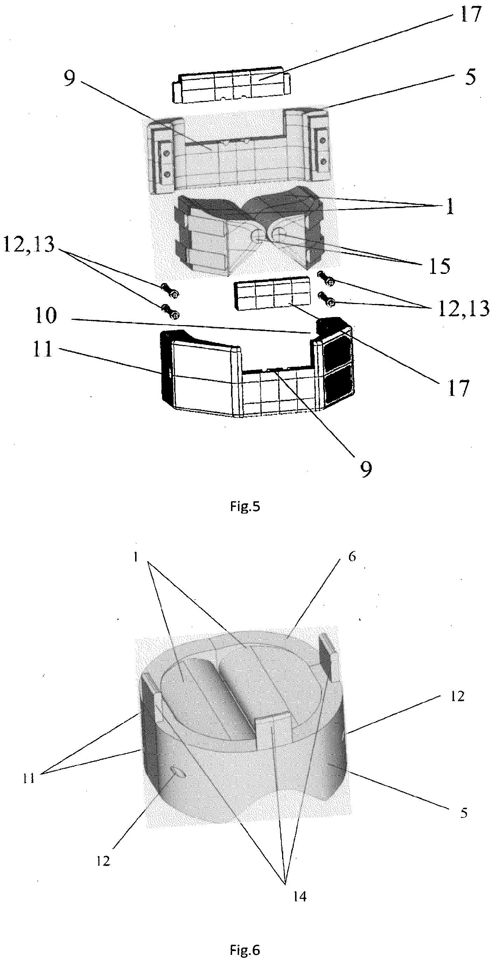

Fig. 4 shows an embodiment of the vocal cord substitution in a different shape for an experimental verification of phonation properties in vitro applicable for placing the substitution into the physiologically defined position of the thyroid cartilage. Fig. 5 shows a disassembled condition of this embodiment. The particular parts and their arrangement corresponds to the vocal cord substitution embodiment as depicted in Figs. 2 and 3, but the walls of guide frame 5 are slanted.

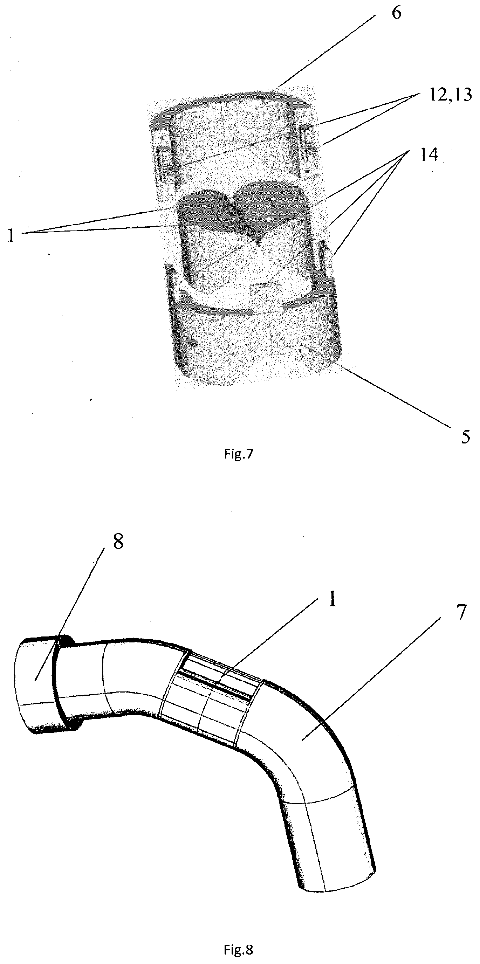

Figs. 6 and 7 show another different embodiment of the vocal cord substitution applicable for the vocal cord substitution implementation in vivo in a place of the tracheal annulus, where both guide frame 5 and setting frame 6 have circular walls equipped with attachment elements 14 for attaching the vocal cord substitution to the human cricoid cartilage.

Fig. 8 shows another embodiment of the vocal cord substitution for its implementation in vivo in tracheostomic cannula 7 fitted with moisturizing and cleaning filter 8 for the inlet air treatment.

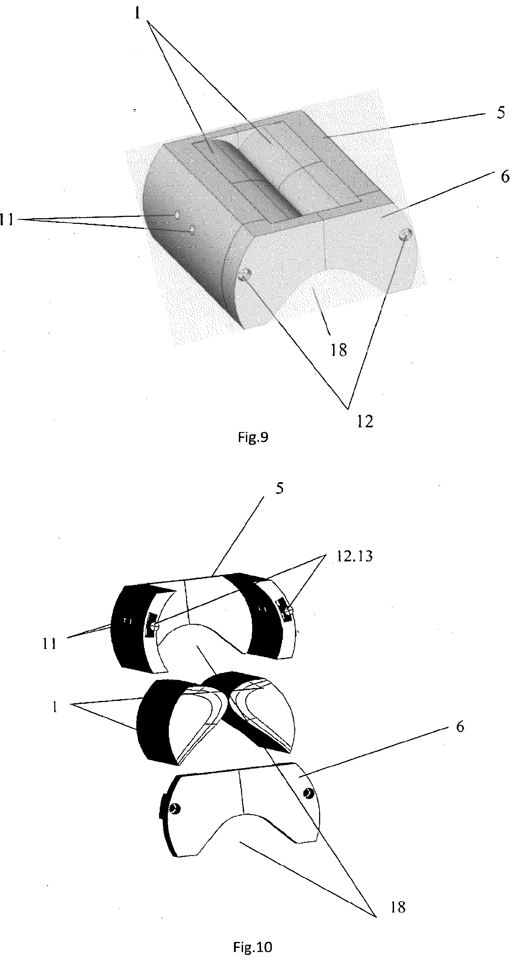

Figs. 9 and 10 show an embodiment of the vocal cord substitution inserted into tracheostomic cannula 7 as depicted in Fig. 8. In this embodiment, which is different in shape, straight-through hole 18 both in setting frame 6 and in guide frame 5 is arranged, enabling the air for breathing to pass through the tracheostomic cannula 7.

In order to set-up the vocal cord substitution for the demanded characteristic of the basic frequency of the vibrating motion of artificial vocal fold 1 the relative distance of attachment walls 9 and guide frame 5 and setting frame 6 can be adjusted, where artificial vocal fold 1 is attached in the longitudinal direction, creating the basic prestressing of artificial vocal fold 1. The demanded vibration characteristic of the artificial vocal cord is achieved using a pressure change in area 3 of artificial vocal fold 1 by connecting area 3 to the pressure fluid source through filling openings 11.

The achieved vibration characteristic is monitored and compared to the demanded vibration physiological characteristics of the human vocal fold. After achieving similarity of the compared vibration characteristics of the artificial vocal fold and the human vocal fold, the vocal cord substitution is tuned for its application.

## Claims

1. A vocal cord substitution comprising an artificial vocal fold, **characterized in that** artificial vocal cord (1) consists of two parts, in the transversal direction the two parts are slidingly placed in opposite sides of guide frame (5) and in the longitudinal direction they are attached to attachment wall (9) of guide frame (5) on one side and to attachment wall (9) of setting frame (6) on the opposite side, whereas inner area (4) of artificial vocal fold (1) is connected to a pressure fluid source.

2. The vocal cord substitution as described in Claim 1, **characterized in that** in guide frame (5) or setting frame (6) an adjusting nut (13) is arranged, into which adjusting bolt (12) is engaging.

3. The vocal cord substitution as described in some of the aforementioned Claims, **characterized in that** artificial vocal fold (1) is fitted with attachment elements (15) on its ends in the longitudinal direction for attaching to attachment walls (9) of guide frame (5) and setting frame (6).

4. The vocal cord substitution as described in Claim 3, **characterized in that** attachment wall (9) of guide frame (5) and attachment wall (9) of setting frame (6) are sectional.

5. The vocal cord substitution as described in some of aforementioned Claims, **characterized in that** artificial vocal fold (1) is fitted with outer layer (2) and middle layer (3) enclosing inner area (4).

6. The vocal cord substitution as described in some of the aforementioned Claims, **characterized in that** guide frame (5) and/or setting frame (6) are fitted with attachment elements (14).

7. The vocal cord substitution as described in some of the aforementioned Claims, **characterized in that** guide frame (5) and setting frame (6) are of rounded prism-shapes, whereas their attachment walls (9) are equipped with straight-through holes (11).

8. A method of tuning the vocal cord substitution comprising an artificial vocal fold as described in Claims 1 - 7, **characterized in that** the basic frequency of the human vocal fold is determined, on the basis of which an initial prestressing of the artificial vocal fold is created by setting a distance of guide frames (5) and setting frames (6) enclosing the artificial vocal fold, followed by changing vibration characteristics of the artificial vocal fold through changing the fluid pressure in the artificial vocal fold; the vibration characteristics are monitored and compared to the demanded vibration characteristics of the human vocal fold, until similarity of the compared vibration characteristics of the artificial and human vocal fold is achieved.
